# EUROPEAN PATENT SPECIFICATION

(11) **EP 3 613 457 B1**
(45) Date of publication and mention of the grant of the patent: **18.02.2026**
(21) Application number: 19191071.0
(22) Date of filing: 09.08.2019
(51) Int. Cl.: A61M 25/00, A61M 25/01

(54) **PACKAGED URINARY CATHETER WITH CATHETER DISPENSING SYSTEM**
VERPACKTER HARNKATHETER MIT KATHETERAUSGABESYSTEM
CATHÉTER URINAIRE EMBALLÉ COMPORTANT UN SYSTÈME DE DISTRIBUTION DE CATHÉTER

(30) Priority: 24.08.2018 US 201816111779
(43) Date of publication of application: 26.02.2020
(73) Proprietor: CONVATEC, INC., Bridgewater, NJ 08807 (US)
(72) Inventor: PALMER, Timothy A., Stillwater, 55082 (US)
(74) Representative: Wilson Gunn

(56) References cited:
- EP-A2- 2 703 036
- FR-A1- 2 794 638
- FR-A5- 2 085 115
- US-A1- 2015 352 324
- US-B1- 9 987 464

## Description

### NOTICE OF COPYRIGHTS AND TRADE DRESS

A portion of the disclosure of this patent document contains material which is subject to copyright protection. This patent document may show and/or describe matter which is or may become trade dress of the owner. The copyright and trade dress owner has no objection to the facsimile reproduction by anyone of the patent disclosure as it appears in the Patent and Trademark Office patent files or records, but otherwise reserves all copyright and trade dress rights whatsoever.

### RELATED APPLICATIONS

The present application is a continuation-in-part of co-pending Patent Application No. 15/671,341, filed August 8, 2017.

### FIELD

The present invention relates to coordinated control devices within a sterile closed intermittent urinary catheter system that facilitate gripping and dispensing of a catheter therein.

### BACKGROUND

People with neurogenic bladder disorders like spinal cord injury, spina bifida or multiple sclerosis, and non-neurogenic bladder disorders like obstruction due to prostate enlargement, urethral strictures or post-operative urinary retention, need to be continuously catheterized to empty their urinary bladders. But such continuous catheterization can lead to problems like urinary tract infections (UTI), urethral strictures or male infertility. Intermittent catheterization at regular intervals avoids such negative effects of continuous long term catheterization. Research has shown that intermittent self-catheterization helps reduce urinary tract infections, control urinary leakage (incontinence) and prevent urinary tract damage.

In our highly mobile culture, the ability to have the freedom to leave home for the day or longer is an important part of life. To accommodate this need single use catheters have been developed to allow patients to perform self-catheterization. Urinary catheters are often lubricated to aid in the insertion into a body cavity, thus making the handling of the catheter difficult and messy. Many catheter packages are now designed with the catheter retained in the package. This allows the user to use the package to manipulate the catheter and avoid the messy and possible unsanitary direct contact with the catheter. For instance, a closed system catheter is a self-contained, sterile, pre-lubricated catheter typically housed within a collection bag which eliminates the need to void the urine into a receptacle or toilet as well as the need to hook up any other kind of bag or container. The closed system is also critical for a sterile intermittent catheter insertion technique whereby the catheter is inserted without human touch. However, manipulating a slippery catheter through a plastic bag can be quite difficult even for someone with excellent dexterity. To aid in the manipulation of the catheter various devices have been conceived to assist in movement of the catheter into and out of its package.

For example, U.S. Patent 9,782,563 to Palmer discloses a package including a bag housing a catheter. The catheter passes through a movement control device retained within a housing at the opening of the bag that allows passage of the catheter out of the bag but resists passage back into the bag. Dispensing of a catheter requires the user to hold the movement control device with one hand while using the other hand to grip the lubricated catheter through the bag, typically by pinching the catheter with the thumb and index finger, and pushing the catheter towards and through the movement control device. The one-way valve function of the movement control device thus aids in dispensing of the catheter. Nevertheless, gripping and pushing of a lubricated catheter through the bag is challenging, particularly so for the elderly and the infirm who are the very people who tend to use urinary catheters.

Accordingly, a substantial need continues to exist for a device capable of facilitating and simplifying dispensing of a sterile closed intermittent urinary catheter.

FR2794638A1 and US9987464B1 provide further examples of prior art catheters.

### SUMMARY OF THE INVENTION

The present application discloses a sterile closed intermittent urinary catheter system which is an easier to use by virtue of coordinated control devices within the system that facilitates gripping and advancement of a catheter therein.

A first aspect of the invention is a packaged catheter equipped with a dispensing system. A first embodiment of the first aspect of the invention includes (i) packaging defining a product retention chamber, (ii) a catheter defining a longitudinal axis retained within the product retention chamber, and (iii) a pair of separately translatable movement control devices, each operably engaging the catheter for permitting unidirectional movement of the catheter in a first axial direction relative to the movement control devices.

A second embodiment of the first aspect of the invention includes (i) packaging defining a product retention chamber, (ii) a catheter retained within the product retention chamber and defining an insertion end, a fixture end and a longitudinal axis, and (iii) first and second separately translatable movement control devices, each operably engaging the catheter for permitting unidirectional movement of the catheter in a first axial direction relative to the movement control devices, wherein (a) pulling the movement control devices away from one another along the longitudinal axis of the catheter effects longitudinal translation of the first movement control device in a second axial direction opposite the first axial direction along the longitudinal length of the catheter with inconsequential longitudinal translation of the second movement control device along the longitudinal length of the catheter, and (b) pushing the longitudinally separated movement control devices towards one another along the longitudinal axis of the catheter effects longitudinal translation of the second movement control device in the second axial direction along the longitudinal length of the catheter with inconsequential longitudinal translation of the first movement control device along the longitudinal length of the catheter.

The first aspect of the invention can optionally be equipped with a handle grip that includes at least (i) a hand-graspable base member fixedly attached to one of the movement control devices, and (ii) a finger-movable member reciprocally engaged to the base member and fixedly attached to the other movement control device, wherein reciprocation of the movable member relative to the base member effects pulling apart and pushing together of the movement control devices along the longitudinal axis of the catheter so as to effect dispensing of the catheter from the packaging.

A second aspect of the invention is a method of dispensing a catheter from a packaged catheter in accordance with the first aspect of the invention wherein the movement control devices permit unidirectional movement of the catheter in a first axial direction relative to the movement control devices. A first embodiment of the second aspect of the invention includes the steps of (i) pulling the pair of movement control devices away from one another along the longitudinal axis of the catheter so as to effect longitudinal translation of a first movement control device in a second axial direction opposite the first axial direction along the longitudinal length of the catheter with inconsequential longitudinal translation of the second movement control device along the longitudinal length of the catheter, and (ii) pushing the longitudinally separated movement control devices towards one another along the longitudinal axis of the catheter so as to effect longitudinal translation of the second movement control device in the second axial direction along the longitudinal length of the catheter with inconsequential longitudinal translation of the first movement control device along the longitudinal length of the catheter, whereby (iii) pushing of the longitudinally separated movement control devices towards one another along the longitudinal axis of the catheter effects dispensing of the catheter out of the packaging. Stated another way, this involves the second movement control device being held stationary against the patient's anatomy while the first movement control device is moved in the first direction to feed the catheter forward. The first movement control device is then moved in the second direction to get another "bite" of the catheter for feed in.

A second embodiment of the second aspect of the invention pertains to dispensing a catheter from a packaged catheter in accordance with the first aspect of the invention which is equipped with a handle grip, and includes the steps of (i) grasping the base member of the handle grip with a first hand, and (ii) reciprocating the button along a path with a finger on the first hand, wherein (a) movement of the button in one direction along the path effects a pulling movement of the pair of movement control devices away from one another along the longitudinal axis of the catheter so as to effect longitudinal translation of a first movement control device in a second axial direction opposite the first axial direction along the longitudinal length of the catheter with inconsequential longitudinal translation of the second movement control device along the longitudinal length of the catheter, and (b) movement of the button in the other opposite direction along the path effects a pushing movement of the pair of movement control devices towards one another along the longitudinal axis of the catheter so as to effect longitudinal translation of the second movement control device in the second axial direction along the longitudinal length of the catheter with inconsequential longitudinal translation of the first movement control device along the longitudinal length of the catheter, effecting a dispensing of the catheter out of the packaging.

### BRIEF DESCRIPTION OF THE DRAWINGS

- Figure 1 is a front view of one embodiment of a packaged catheter in accordance with this invention, depicting an assembly of a pair of movement control members of a dispensing system pulled apart in a longitudinally spaced relationship and the catheter fully retained within the packaging.
- Figure 2 is a front view of the invention depicted in Figure 1 depicting the movement control devices of the dispensing system pushed together and the catheter partially dispensed from the packaging.
- Figure 3 is a perspective view of one of the movement control devices depicted in Figures 1 and 2 with the locking member pivoted into the second locking position.
- Figure 4 is a side view of the catheter movement control device depicted in Figure 3.
- Figure 5 is an end view of the catheter movement control device depicted in Figure 3 .
- Figure 6 is a cross-sectional side view of the catheter movement control device depicted in Figure 3 taken along line 6-6.
- Figure 7 is a cross-sectional side view of the catheter movement control device depicted in Figure 3 taken along line 7-7, but with the locking mechanism pivoted into a first dispensing position.
- Figure 8 is a front view of a portion of another embodiment of a packaged catheter in accordance with this invention schematically depicting a handle grip component for facilitating single-handed dispensing of the catheter.
- Figure 9 is a front view of a portion of the handle grip component schematically depicted in Figure 8 , further equipped with a schematically depicted release element.
- Figures 10A-10C show a pair of movement control devices mounted over a catheter to illustrate a sequence of movement of the catheter using coordinated manipulation of the control members.
- Figure 11 is a plan view of an integrated assembly of a pair of movement control devices mounted within a handle grip component for facilitating single-handed dispensing of the catheter.
- Figure 12 is a perspective view of an integrated assembly of a pair of movement control devices within relatively sliding housings, while Figure 13 is an exploded plan view thereof.
- Figures 14A-14D are various views of an outer base member that forms a part of the integrated assembly of Figure 12, and Figures 15A-15D are various views of a smaller movable member that slides within the base member.
- Figure 16A is a front view of one embodiment of a packaged catheter having a sterile bag and a pair of movement control devices mounted therein for advancing a catheter, and a safety device in the form of a third movement control device mounted near an outlet that prevents premature distal movement of the catheter from the sterile bag, and Figure 16B is an enlargement of the outlet showing operation of the safety device.
- Figure 17A is a front view of one embodiment of a packaged catheter having a sterile bag and an integrated assembly of a pair of movement control devices mounted therein for advancing a catheter, as well as a safety device in the form of a third movement control device mounted near an outlet that prevents premature distal movement of the catheter from the sterile bag, and Figure 17B is an enlargement of the outlet showing operation of the safety device.
- Figure 18 is a perspective view of an integrated assembly of a pair of movement control devices within relatively sliding housings having an alternative finger pad.
- Figures 19A-19D are perspective views of the integrated assembly of Figure 18 mounted to an outer panel of a sterile bag with alternative friction-enhancing pads and straps in operative relationship with the finger pad.
- Figures 20A-20D are perspective views of the integrated assembly of Figures 19A and 19C mounted to an outer panel of a sterile bag showing a variety of different ways to grasp and reciprocate the finger pad to advance the catheter from within the bag.

### DETAILED DESCRIPTION OF A PREFERRED EMBODIMENT

The present application provides a sterile closed intermittent urinary catheter system which is easier to use by virtue of coordinated control devices within a sterile bag that facilitates gripping for advancement and retrieval of the catheter from the collection bag. The catheter is advanced by hand through manipulation from outside the sterile bag.

### Nomenclature

- **10** Packaged Catheter
- **20** Packaging
- **21** First Longitudinal End of Packaging
- **22** Second Longitudinal End of Packaging
- **29** Product Retention Chamber
- **30** Dispensing System
- **31** First Movement Control Device
- **32** Second Movement Control Device
- **40** Main Body of Each Movement Control Device
- **401** First Portion of Main Body of Each Movement Control Device
- **402** Second Portion of Main Body of Each Movement Control Device
- **40LA** Longitudinal Axis of Main Body
- **41** First Longitudinal End of Main Body
- **42** Second Longitudinal End of Main Body
- **43** Engagement Members
- **431** First Longitudinally Extending Engagement Member
- **432** Second Longitudinally Extending Engagement Member
- **45** Cap or Seal snap fit on the Main Body to hold the soft silicone introducer tip in place at the exit
- **49** Passageway Through Main Body
- **49i** Interior End (Opening) of Passageway Through Main Body
- **49e** Exterior End (Opening) of Passageway Through Main Body
- **49CA** Central Axis of Passageway
- **50** Locking Member of Each Movement Control Device
- **51** First or One Lateral End of Locking Member
- **52** Second or Other Lateral End of Locking Member
- **55** Hinge
- **55P** Hinge Pivot Axis
- **59** Orifice Though Locking Member
- **59CA** Central Axis of Orifice
- **60** Catheter
- **61** Lumen or Insertion End
- **62** Funnel or Fixture End
- **69CA** Longitudinal Central Axis of Catheter
- **69x1** First Axial Longitudinal Direction
- **69x2** Second Axial Longitudinal Direction
- **70** Handle Grip
- **71** Base Member of Handle Grip
- **72** Activation Element or Button on Handle Grip
- **72p** Path of Movement of Activation Element or Button
- **80** Release Actuator Element
- **90** Handle Opening in Packaging
- **X** Longitudinal Direction
- **Y** Lateral Direction
- **Z** Transverse Direction

### Definitions

As utilized herein, including the claims, the term **"inconsequential,"** when used to describe longitudinal translation of a movement control device along the longitudinal length of a catheter, means a distance of less than 1 cm.

### CONSTRUCTION

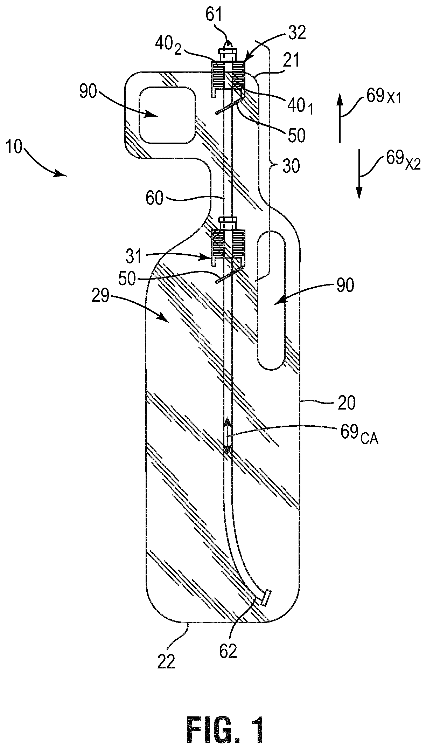

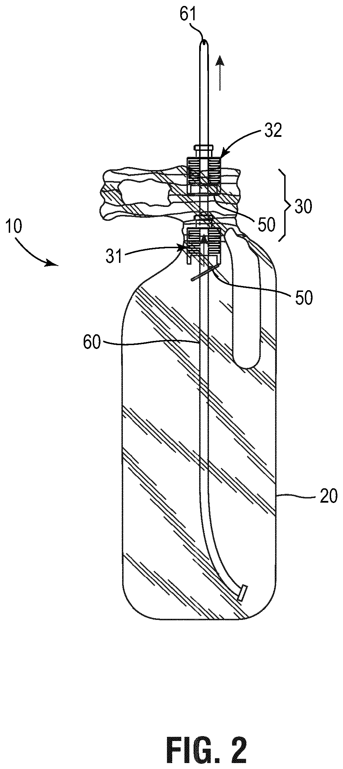

Referring to Figures 1 and 2 , the invention is a packaged catheter **10** equipped with a dispensing system **30.**

### PACKAGED CATHETER

The packaged catheter **10** includes a catheter 60 , such as an intermittent urinary catheter, retained within the product retention chamber **29** of a package **20.** The catheter **60** defines an insertion end **61** and a fixture end **62 ,** and a longitudinal central axis **69CA.** Consistent with similar devices, advancement of the catheter **60** into the urethra occurs in a distal direction and retraction in a proximal direction. Therefore, the insertion end **61** is the distal end and the fixture end **62** is the proximal end.

### CATHETER

The catheter **60** may have any desired longitudinal length and shape effective for achieving the function of eliminating urine from the bladder of a male or female patient. Preferably, the longitudinal length for an adult female catheter **60** is between 2-6 inches, the longitudinal length of the adult male catheter **60** is between 10-16 inches, and the longitudinal length of a pediatric catheter **60** is between 5-11 inches (127-279 mm).

### PACKAGING

The packaging 20 may be selected from any of the customary packaging used for catheters so long as the packaging is sufficiently supple and flexible that the packaging **20** does not prevent or inhibit translation of the movement control devices ( **31** and **32** ) towards and away from one another when gripped through the packaging **20.** In a preferred embodiment, the packaging **20** is in the form of a polymer bag sealed around its exterior edges and around any handle openings therein. The packaging **20** will thus be referred to as a bag from now on, but one of skill in the art will understand that alternative packaging solutions are possible. For instance, the term "bag" implies a closed end, while the packaging used in the catheter system disclosed herein may not be closed, and may instead define a drain line therethrough.

### DISPENSING SYSTEM

The dispensing system **30** includes a pair of movement control devices ( **31** and **32** ) for facilitating longitudinal **x** movement of the catheter **60** from a second longitudinal end **22** of the bag **20** towards a first longitudinal end **21** of the bag **20** for controlled dispensing of the catheter 60 from the bag **20.**

The movement control devices ( **31** and **32** ) each operably engage the catheter **60** and are separately translatable along the longitudinal central axis **69CA** of the catheter **60** to cause unidirectional movement of the catheter **60** in a first axial direction **69x1** (distally) relative to the movement control devices ( **31** and **32** ) . More particularly, and as will be explained below, each movement control device ( **31** and **32** ) is constructed so as to automatically be able to freely slide along the longitudinal length of the catheter **60** in a second axial direction **69x2** , but is automatically unable to freely slide along the catheter **60** in the first axial direction **69x1.**

The unidirectional nature of the movement control devices ( **31** and **32** ), allows a user to quickly, easily and controllably dispense a catheter **60** from a bag 20 by repetitively pushing together and pulling apart the paired set of movement control devices ( **31** and **32** ). In a preferred embodiment, an outer movement control device **32** is secured to the first longitudinal end **21** of the bag **20** and may be partially exposed out of the first longitudinal end **21**, while the inner movement control device **31** is contained within the bag **20.** In the configuration of Figure 1 , the inner movement control device **31** is spaced apart from the outer movement control device **32 ,** while in Figure 2 the inner movement control device **31** has been pushed towards the outer movement control device **32.** The catheter **60** is pushed by the inner movement control device **31** which resists relative movement of the catheter therethrough in the second axial direction **69x2,** but the catheter slides easily in the first axial direction **69x1** (distally) through the outer movement control device **32.** Subsequently, the inner movement control device **31** may be displaced longitudinally over the catheter **60** in the second axial direction **69x2** (proximally) and away from the outer movement control device **32 ,** back into the position shown in Figure 1.

To summarize, pushing the longitudinally separated movement control devices ( **31** and **32** ) towards one another along the catheter **60** effects longitudinal translation of the second movement control device **32** in the second axial direction **69x2** with inconsequential longitudinal translation of the first movement control device **31** along the catheter **60.** Conversely, pulling the movement control devices ( **31** and **32** ) away from one another along the longitudinal length of the catheter **60** effects longitudinal translation of the first movement control device **31** along the catheter **60** in the second axial direction **69x2** with inconsequential longitudinal translation of the second movement control device **32** along the catheter **60.** Stated another way, displacing the inner movement control device **31** in a distal direction relative to the second movement control device **32** advances the catheter **60** , and retracting the inner movement control device **31** in a proximal direction relative to the second movement control device **32** resets the inner control device **31** to its initial position without moving the catheter **60.**

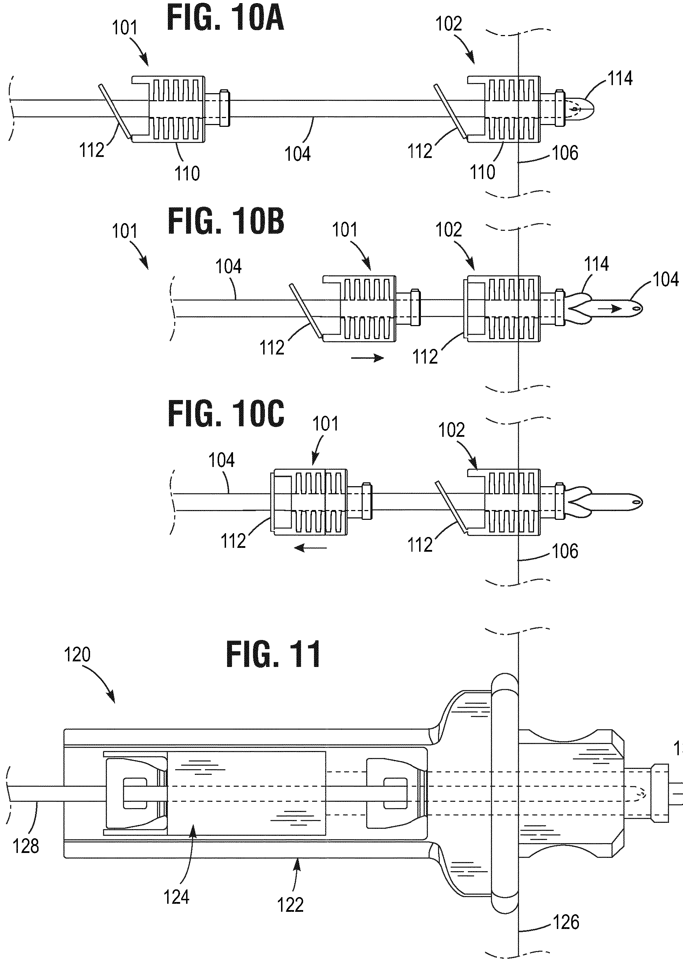

A further explanation and illustration of the coordinated manipulation of a paired set of movement control devices is provided below in the context of Figures 10A-10C .

### MOVEMENT CONTROL DEVICE

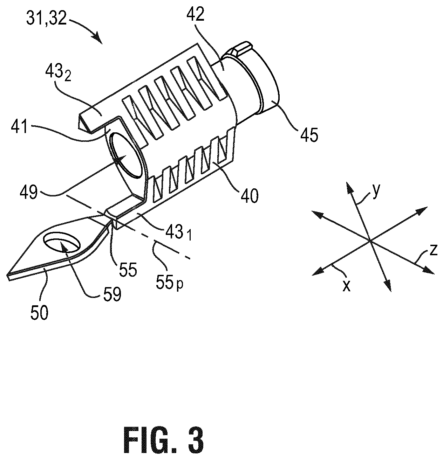

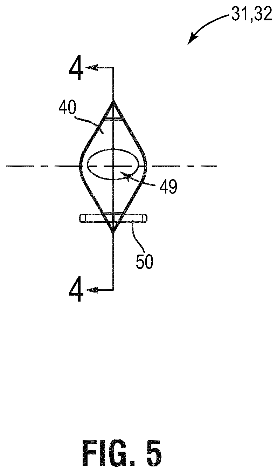

Figures 1-9, and in particular Figures 3-7 , depict a preferred embodiment of the movement control devices **31** , **32**.

This embodiment of the movement control device **31** , **32** has a main body **40** and a locking member **50** hingedly attached to the main body **40.** The main body **40** and locking member **50** are preferably formed as a monolithic device with the locking member **50** pivoting about a living hinge **55** formed in the single piece device. The locking member **40** may be made from any suitable material, including various plastics such as polyethylene, polypropylene, polyvinyl chloride (PVC), and nylon.

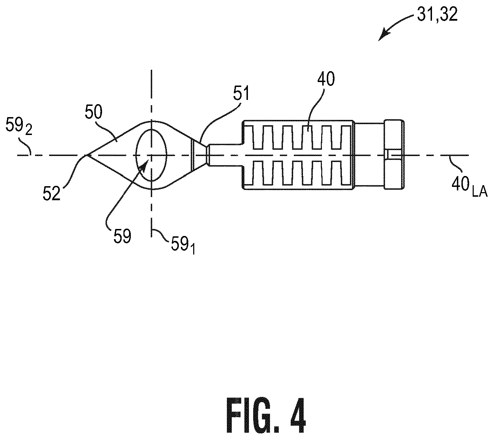

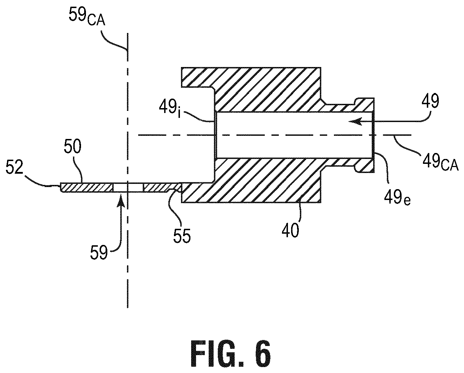

The main body **40** of the movement control device **31** , **32** has a first longitudinal end **41** and a second longitudinal end **42 ,** and defines a longitudinal axis **40LA** ( Figure 4 ). A passageway **49** extends through the main body **40** from an opening **49i** in the first longitudinal end **41** of the main body **40** to an opening **49e** in the second longitudinal end **42** of the main body **40.** The passageway **49** is preferably linear and defines a central axis **49CA** ( Figure 6 ). The passageway **49** is sized and configured to allow passage of the lumen portion of a catheter **60.**

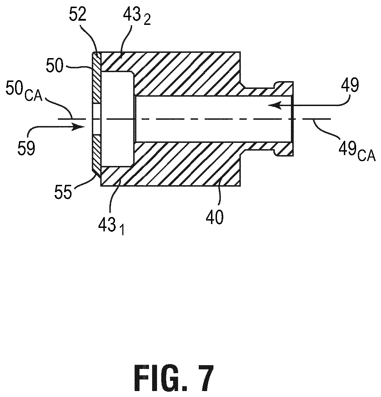

A first lateral end **51** of the locking member **50** hingedly attaches to the main body **40** at hinge **55** , permitting pivoting of the locking member **50** relative to the main body **40** about a hinge pivot axis **55p** between a first aligned position depicted in Figure 7 , and a second misaligned position depicted in Figures 3-6 . In the first aligned position the second lateral end **52** of the locking member **50** contacts the main body **40** and the central axis **59CA** of an orifice **59** through the locking member **50** is aligned with the central axis **49CA** of the passageway **49.** When in the first aligned position a catheter **60** may be axially translated through the aligned orifice **59** and passageway **49.** Pivoting of the locking member **50** from the first aligned position towards the second misaligned position pivots the second lateral end **52** of the locking member **50** away from the main body **40 ,** resulting in an increasing misalignment of the central axis **59CA** of the orifice **59** and the central axis **49CA** of the passageway **49** until movement of a catheter **60** is inhibited through the misaligned orifice **59** and passageway **49.** Stated another way, friction between the catheter **60** and the orifice **59** pivots the locking member **50** away from the main body **40** into an angled position such that the catheter binds within the orifice **59** and is prevented from moving farther.

In a preferred embodiment, laterally **y** spaced engagement members **43** extend longitudinally **x** from a longitudinal end of the main body **40 ,** with a first lateral end **51** of the locking member **50** hingedly attached to a first engagement member **431** at hinge **55 ,** and the second engagement member **432** located to contact the second lateral end **52** of the locking member **50** when the locking member **50** is in the first aligned position. The engagement members **43** provide a modest offset between the passageway **49** and the orifice **59** for avoiding severe bending and kinking of the catheter **60** when the central axis **59CA** of the orifice **59** and the central axis **49CA** of the passageway **49** are misaligned.

Pivoting of the locking member **50** about the hinge pivot axis **55p** is effected by axial translation of the movement control device **31 , 32** along the longitudinal length of a catheter **60** passing through the passageway **49** and frictionally passing through the orifice **59** in the movement control device **31** , **32.** Referring generally to Figures 1 and 2, axial translation of a movement control device **31** , **32** in the first longitudinal direction **69x1** along the length of a catheter **60** causes pivoting of the locking member **50** towards the second misaligned position so as to lock the movement control device **31** , **32** onto the catheter **60.** When locked, any further movement of the movement control device **31** , **32** in the first longitudinal direction **69x1** will effect concomitant movement of the catheter 60 along with the movement control device **31** , **32** in the first longitudinal direction **69x1.** In other words, the movement control device **31** , **32** pushes the catheter **60.**

In contrast, axial translation of a movement control device **31** , **32 in** the second longitudinal direction **69x2** along the length of a catheter **60** causes pivoting of the locking member **50** towards the first aligned position so as to unlock the movement control device **31** , **32** from the catheter **60.** When unlocked, the movement control device **31** , **32** is free to travel along the longitudinal length of the catheter **60.** Such freedom of travel can continue along the entire length of the catheter **60** in the second longitudinal direction **69x2,** but will of course be promptly lost when the movement control device **31, 32** is moved in the first longitudinal direction **69x1** as the locking member **50** will pivot into the second misaligned position and lock the movement control device **31** , **32** onto the catheter **60.**

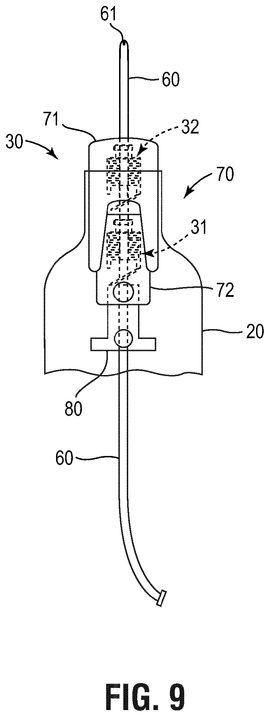

It should be noted that the friction between the catheter **60** and the orifice **59** of each movement control device **31** , **32** occurs any time there is relative movement therebetween; i.e., automatically. Therefore, if the catheter **60** slides distally through the orifice **59** it contacts the inner edges of the orifice and causes the locking member **50** to pivot to an aligned unlocked position, whereas when the catheter **60** slides proximally through the orifice **59** it almost immediately causes the locking member **50** to pivot to a misaligned or locked position which binds on the catheter **60.** In other words, the catheter **60** may be advanced through each movement control device **31** , **32** but cannot be retracted. However, provision may be made to bypass the locking member **50** by including a latch or some other physical impediment to its free movement; such as latching the locking member **50** in an unlocked position. In one example, a release actuator element **80** is shown in and described with respect to Figure 9 .

The size and dimensions of the movement control device **31** , **32** are generally dictated by the size of the catheter **60** with which it is used, but the main body **40** should be large enough to be retentively pinched between the thumb and index finger in order to allow dispensing of the catheter **60** from the bag **20** through the movement control device **31 , 32**. Dimensions of an exemplary movement control device **31** , **32** are provided in Table One below.

| | |
|---|---|
| Longitudinal Length of Main Body **40** | 25 mm |
| Lateral Width of Main Body **40** | 20 mm |
| Transverse Depth of Main Body **40** | 10 mm |
| Cross Sectional Area of Passageway **49** | 200 mm2 |
| Thickness of Locking Member **50** | 1-2 mm |
| Cross Sectional Area of Orifice **59** | 100 mm2 |

Referring again to Figures 1 and 2, at least one of the movement control devices **31** and **32** is preferably fixedly attached to the bag **20** when incorporated into a packaged catheter **10.** Namely, a first portion **401** of the main body **40 of the outer** movement control device **32** is positioned within the product retention chamber **29** defined by the bag **20 ,** and a second portion **402** of the main body **40** is positioned exterior to the product retention chamber **29.** The passageway **49** through the main body **40** of the movement control device **32** provides a port through the bag 20 from an interior end **49i** of the passageway **49** to an exterior end **49e** of the passageway **49.** The affixed movement control device **32** can conveniently be heat sealed at a longitudinal end **21** of the bag **20.**

As seen in Figure 3 , a cap or seal **45** can be placed over the exterior end **49e** of the passageway **49** to maintain sterility prior to usage. The inner movement control device **31** is preferably wholly located within the product retention chamber **29** of the bag **20** and may also be fixedly attached to the bag **20** so long as the bag **20** is supple enough to allow the movement control devices **31** and **32** to be pushed together and pulled apart.

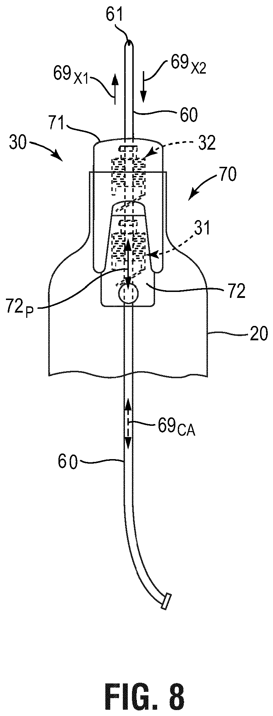

The dispensing system **30** may further include a handle grip **70** as seen in Figure 8 for facilitating single-handled dispensing of the catheter **60** from the bag **20** using the dispensing system **30 ,** a feature long sought by users.

Figure 8 schematically depicts the handle grip **70** having a hand-graspable base member **71** , and a finger-movable member **72** reciprocally (i.e., telescopically) engaged to the base member **71** for travel along a path **72p** relative to the base member **71.** The base member **71** and movable member **72** are preferably sized, configured and arranged for thumb actuation of the movable member **72** while cradling of the base member **71** within the palm of that hand. Of course, the term "finger-movable" implies that the member **72** is movable by a finger, thumb, fist, wrist or any other part of the hand or arm, as well as by using intermediate inanimate objects. The base member **71** is fixedly attached to a second outer one of the movement control devices **32 ,** while the movable member **72** is fixedly attached to a first inner one of the movement control devices **31** , whereby reciprocation of the movable member **72** relative to the base member **71** along a path **72p** effects a pulling apart and pushing together of the movement control devices **31** and **32** along the longitudinal axis **69CA** of the catheter **60** so as to effect dispensing of the catheter **60** from the bag **20.**

The finger-movable member **72** may optionally be biased, such as by use of a spring, towards the pushed apart configuration to effect auto "reloading" of the dispensing system **30.** When the dispensing system **30** includes a handle grip **70 ,** the bag **20** can be conveniently heat sealed to the base member **71** with the movable member **72** retained wholly within and actuated through the bag **20.**

Referring to Figure 9 , a release actuator element **80** may optionally be provided for effecting selective manual sustained pivoting of the locking member **50** on the first movement control device **31** into the aligned position regardless of real-time longitudinal movement of the catheter **60** relative to the first movement control device **31.** This permits movement of a dispensed catheter 60 in the second axial longitudinal direction **69x2** (i.e., retraction) relative to the first movement control device **31.** Specifically, actuating the release actuator element **80** while at the same time pushing the first movement control device **31** against the second movement control device **32** to pivot its locking member **50** into the aligned position enables retraction of the catheter **60** back into the bag **20.** At the same time, the release actuator element **80** also pushes the first movement control device **31** against the locking member **50** of the second movement control device **32 ,** thus holding the second locking member open to allow free movement through both movement control devices.

The release actuator element **80** may be telescopingly mounted onto the movable member **72** for travel between a first disengaged position and a second engaged position. In the disengaged position, the release actuator element **80** is spaced from the locking member **50** on the first movement control device **31**, while in the engaged position the release actuator element **80** contacts and pivots the locking member **50** on the first movement control device **31** into an aligned positioned.

### USE

The packaged intermittent urinary catheter **10** can be used by patients for self-catheterization. Prior to use the patient should take all sanitary procedures advised by their doctors to decrease the risks of infection.

Referring to the embodiment depicted in Figures 1 and 2 , first the seal or cap **45** is removed to open the port through the bag **20** defined by the passageway **49** through the main body **40** of the second movement control device **32.**

The user then grasps or pinches the main body **40** of the first or inner movement control device **31** through the bag **20** with one hand, grasps or pinches the main body **40** of the second or outer movement control device **32** with the other hand through the bag **20** , and then repetitively pushes together and pulls apart the movement control devices **31** and **32** as depicted in Figures 1 and 2. As mentioned, pulling the two movement control devices **31** and **32** apart "loads" the dispensing system **30** without expelling the catheter **60** from the bag **20 ,** and pushing the movement control devices **31** and **32** together dispenses a length of the catheter **60** from the bag **20.**

A clearer depiction of this "inchworm" sort of catheter advancement is seen in Figures 10A-10C where a first or inner movement control device **101** and a second or outer movement control device **102** are shown mounted over a catheter **104.** The first and second movement control devices **101 , 102** may be identical to that shown in Figures 3-7 . By "mounted over" the catheter **104** is meant that the catheter passes through the longitudinal passageway through the main body **110** and the orifice though the locking member **112** of each movement control device **101 , 102.** A symbolic line **106** is drawn to represent an outer edge of the sterile bag within which the catheter **104** is stored.

Figure 10A is a resting position with the catheter **104** stored within the sterile bag **106** and preferably inside of a bullet-shaped introducer tip **114** mounted to an exterior end of the outer movement control device **102.** The introducer tip **114** is sized to fit within the outer end of the urethra and made of a flexible elastomer which has petals that the catheter **104** spreads apart upon passage therethrough. When the user wishes to utilize the catheter, the bag **106** is first brought into proximity with the genitals, and the introducer tip **114** inserted into the tip of the urethra. The introducer tip **114** helps prevent any bacteria that may be around the urethra opening from contacting the catheter **104 ,** which in turn helps reduce instances of infection.

Next, the user holds the outer movement control device **102** steady through the bag **106** and grasps and advances it toward the inner movement control device **101 ,** as in Figure 10B. Friction between the catheter **104** and the orifice though the locking member **112** of the inner movement control device **101** pivots the locking member away from the main body **110** to about a 45° angle as shown. This misaligns the orifice though the locking member **112** with the catheter axis and causes the catheter **104** to be pushed along by the inner movement control device **101.** The catheter **104** emerges from the petals of the introducer tip **114** into the outer end of the urethra. Friction between the catheter **104** and the orifice though the locking member **112** of the outer movement control device **102** forces the locking member to pivot toward and eventually contact the main body **110** , which aligns the orifice though the locking member **112** with the catheter axis and permits catheter movement.

Finally, Figure 10C shows leftward movement of the inner movement control device **101** over the catheter **104** which pivots its locking member **112** against the main body **110 ,** aligns the orifice though the locking member with the catheter axis and permits movement thereover. The locking member **112** of the outer movement control device **102** is slightly pulled by the catheter **106** to the angled, misaligned position shown, which creates friction on the catheter and prevents it from being retracted farther back into the bag **106.** Repeating the steps shown in Figures 10B and 10C gradually inches the catheter **104** out of the sterile bag **106** and through the urethra of the user to the desired location where urine flows.

Referring to Figures 1 and 2 , two-handed pushing and pulling of the movement control devices **31** and **32** to effect dispensing of a catheter **60** may be simplified by attaching both movement control devices **31** and **32** to the bag **20** in longitudinally spaced relationship and providing a handle opening **90** proximate each movement control device **31** and **32.**

The dispensing system **30** also functions to preventing the fixture end **62** of the catheter **60** from advancing out of the bag **20.**

Referring to the embodiment depicted in Figures 8 and 9 , the user cradles the base member **71** in the palm of a hand with the fingers wrapped around and gripping the base member **71** and the thumb of that same hand placed upon a pad on the activation element **72.** The user then uses the thumb to reciprocate the activation element **72** relative to the base member **71** along the path of travel **72p** , thereby pulling the movement control devices **31** and **32** apart and loading the system. Subsequently, pushing the movement control devices **31** and **32** together dispenses a length of the catheter **60** from the bag **20** when the activation element **72** is moved in the opposite direction along the path **72p.**

Still referring to Figures 8 and 9 , one-handed pushing and pulling of the movement control devices **31** and **32** to effect dispensing of a catheter **60** may be simplified by including the handle grip 70 feature.

### ALTERNATIVE EMBODIMENTS

In addition to the previously-described movement control devices and handle grips integrating the same, the applicants have developed further alternatives for use in sterile closed intermittent urinary catheter systems, as explained below.

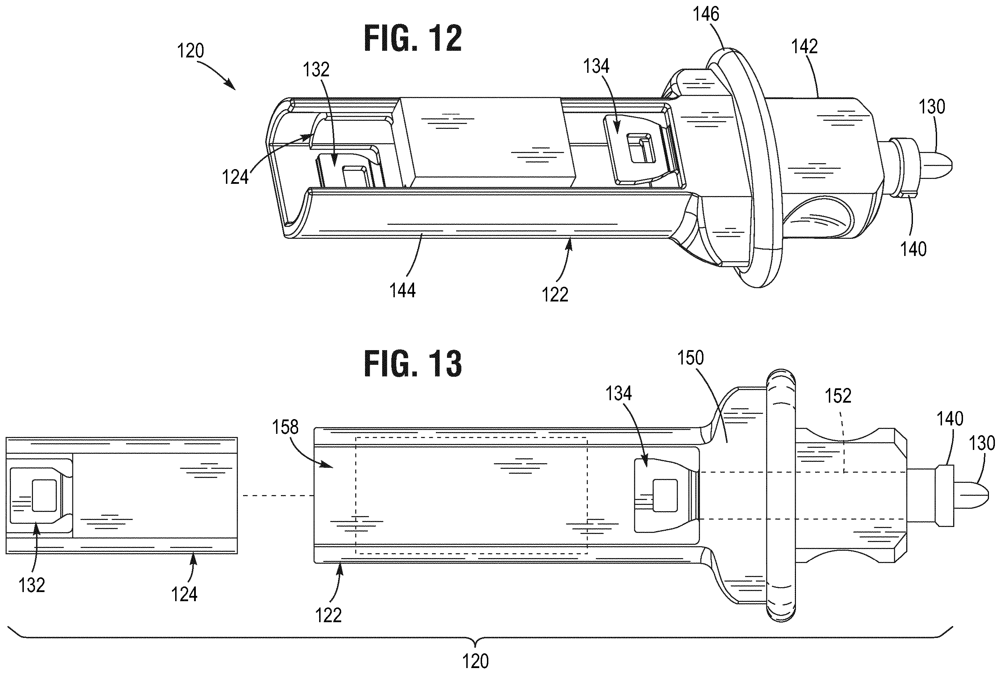

Figure 11 is a plan view of an integrated assembly **120** of a pair of movement control devices mounted within a handle grip for facilitating single-handed dispensing of the catheter. With specific reference to Figures 12-15 , the integrated assembly **120** includes a larger base member **122** and a smaller movable member **124** arranged to move within the base member. The base member **122** is desirably secured to one end of a sterile bag, as schematically shown at **126** in Figure 11 , and defines an exterior handle grip of the integrated assembly **120.** A catheter **128** passes longitudinally through the assembly **120** and typically is positioned with its distal tip just inside of an introducer tip **130** in a stored position.

Greater detail of the integrated assembly **120** is shown in Figures 12-15 , but Figure 11 depicts the sterile bag **126** at the same placement as the sterile bag 106 in Figures 10A-10C as well as a pair of movement control devices **132, 134** within the assembly. A first or inner movement control device **132** forms a part of the movable member **124** while a second or outer movement control device **134** forms a part of the base member **122.** It will be understood that relative displacement of the movement control devices **132 , 134** advances the catheter **128** in the "inchworm" manner depicted in Figures 10A-10C .

With reference now to the detailed views of Figures 12-15 , the larger base member **122** includes a distal hub **140** from which the introducer tip **130** projects. The hub **140** in turn is molded together with an ergonomic external handle **142** and an internal housing **144** which are separated by a flange **146.** When assembled with the sterile bag **126**, the handle **142** is outside while the housing **144** is inside, with the bag **126** preferably adhered or heat sealed to the exterior of the flange **146 ,** as seen in Figure 11 . The integrated assembly **120** has a generally rectangular lateral cross-section which presents a flat nonrotating profile inside the bag to help the user apply pressure from both sides.

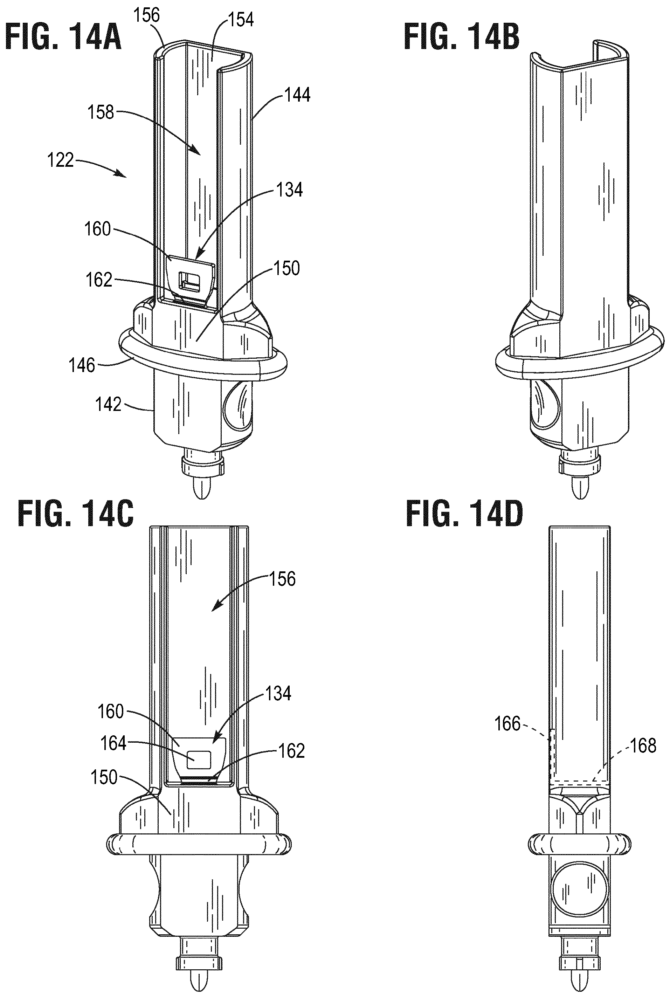

The internal housing **144** includes a relatively wide distal end **150** completely surrounding a lumen **152** therethrough, shown in Figure 13 , that also passes through the external handle **142** and distal hub **140** for passage of the catheter **128.** On its proximal end, the housing **144** includes an elongated chute that extends into the bag **126.** The chute is closed on three sides by a floor **154** and two sidewalls **156** that are slightly concave on their inner faces. The chute defines a longitudinal slightly oval channel **158** therein for reciprocal movement of the movable member **124.** The movable member **124** is shown within the channel **158** in Figure 12 and exploded therefrom in Figure 14, with an outline thereof in phantom the channel **158.**

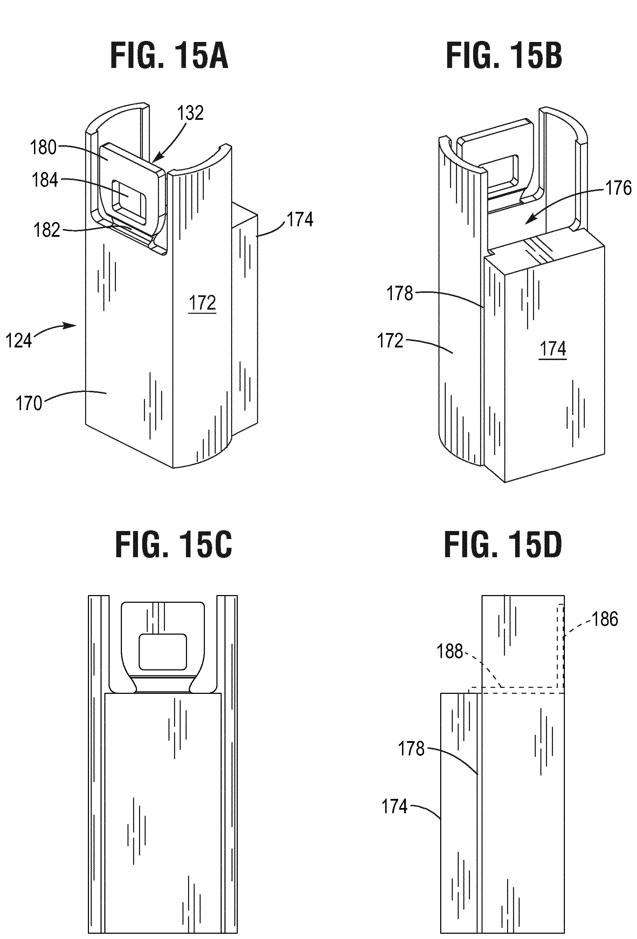

The second or outer movement control device **134** comprises a cantilevered tab 160 connected to one side of the wide distal end **150** at a hinge **162** , preferably a living hinge. Instead of a discrete main body, as with the movement control devices described above, the rest of the base member **122** serves as the main body. The tab **160** has an orifice **164** therethrough that serves to alternately slide over or catch on the catheter **128.** The tab **160** generally bends from the longitudinally-aligned position **166** as shown in Figures 12 and 15D down into the channel **158** into engagement with the catheter **128** and to a misaligned position **168** angled about 90° from longitudinal.

As seen in Figures 14A-14D , the movable member **124** has a generally tubular housing formed by a floor **170 ,** two sidewalls **172** and an upper finger pad **174.** The two sidewalls **172** are slightly outwardly convex so as to closely fit within the slightly oval channel **158** defined by the chute of the base member **122.** The housing defines a longitudinal throughbore **176** for passage of the catheter **128.** The upper finger pad **174** is separated from the two sidewalls **172** by a pair of outwardly-directed longitudinal grooves **178** whose purpose will be explained below.

The first or inner movement control device **132** comprises a cantilevered tab **180** connected to the floor **170** at one side of the generally tubular housing with a hinge **182 ,** preferably a living hinge. Instead of a discrete main body, as with the movement control devices described above, the rest of the movable member **124** serves as the main body. The tab **180** has an orifice **184** therethrough that serves to alternately slide over or catch on the catheter **128.** The tab **180** generally bends from the longitudinally-aligned position **186** as shown in Figures 12 and 14D up into the throughbore **176** into engagement with the catheter **128** and to a misaligned position **188** angled about 90° from longitudinal. As seen best in Figure 14B , the finger pad **174** terminates short of the two sidewalls **172** so that the tab **180** contacts one end of the finger pad **174** when in the misaligned position **188.**

Now with reference back to Figures 12 and 13 , the movable member **124** fits closely within the slightly oval channel **158** defined by the chute of the base member **122 ,** with the first or inner movement control device **132** on an end opposite the second or outer movement control device **134.** The outwardly-directed longitudinal grooves **178** flanking the upper finger pad **174** receive longitudinal edges of the two sidewalls **156** on the chute of the base member **122** so that the movable member **124** is held and guide thereby.

To assemble the sterile closed intermittent urinary catheter system, the movable member **124** is first inserted into the oval channel **158** of the base member **122.** Next, the two tabs **160** , **180** forming the movement control devices **132** , **134** are bent so that they extend across the respective passageways defined by the base member **122** and the movable member **124.** Once bent, the catheter **128** may pass through the orifices 164, 184 of the tabs **160 , 180** and positioned with its distal tip just inside the introducer tip **130** , as seen in Figure 11 . The outer sterile bag **126** may be formed around the assembly **120 ,** preferably after positioning of the catheter **128** therein.

When assembled, the user may easily manipulate the finger pad **174** through the flexible plastic of the bag **126** while simultaneously holding still the base member **122 ,** such as by grasping the ergonomic external handle **142.** This operation may even be done with one hand. To help move the finger pad **174** , rubber tape or other such friction-inducing material may be added to the exterior of the bag over the pad, and various other solutions are disclosed herein. The generally rectangular lateral cross-section of the integrated assembly **320** aids in these various movements by presenting resistance to rotation about the longitudinal axis and also a flatter profile for the user to compress between his or her hands.

### FEED LOCK

In addition to facilitating advancement of a catheter from a closed intermittent urinary catheter system, the present application also discloses a safety measure to help prevent premature expulsion of the catheter from the sterile bag. As with most such systems, once an outer packaging is removed, the catheter may be advanced. If the user has not yet positioned and inserted an introducer tip into the urethra, there is the possibility that the catheter may be prematurely advanced and then come into contact with the exterior of the urethra, an area with many germs. If the user inadvertently picks up bacteria from outside the urethra and transfers it into the urethra, an infection may ensue.

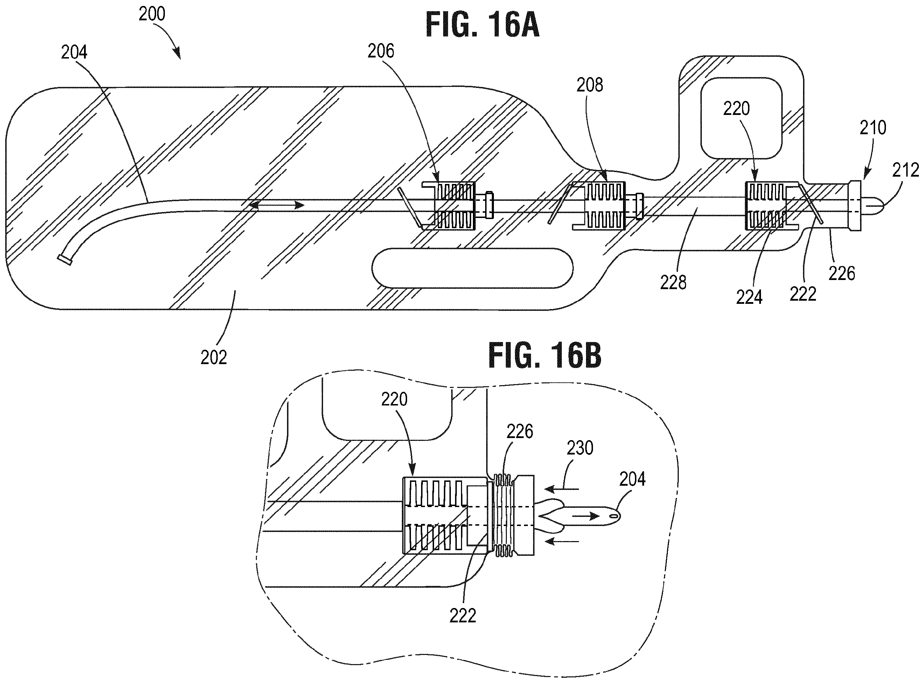

Figures 16 illustrates a system of a sterile catheter package **200** having a flexible bag **202** defining a reservoir therein and containing a catheter **204** and a dispensing system with a first movement control device **206** and a second movement control device **208.** The package **200** and first and second movement control devices **206 , 208** are as described above, with the latter facilitating advancement of the catheter **204** from within the reservoir and out through an outlet **210** which may include an introducer tip **212.**

The system further includes a safety device in the form of a third movement control device **220** mounted over the catheter **204** adjacent its distal tip. Unlike the other two, the orientation of the third movement control device **220** is reversed with a locking tab **222** located on a distal side of a main body **224.** The third movement control device **220** is mounted close to the outlet **210** and in particular just inside a collapsible extension **226** of the bag **202.** The locking tab **222** has an orifice through which the catheter **204** extends and when the catheter **204** is displaced distally relative to the third movement control device **220** it tends to pivot the locking tab **222** away from the main body **224** such that the orifice becomes misaligned with the catheter axis - the locked position. Thus, in the configuration shown in Figure 16 the third movement control device **220** prevents distal movement of the catheter **204** and thus prevents the catheter from being dispensed from within the bag **202.**

To operate the system, the user first inserts the introducer tip **212** into the urethra and applies pressure by manipulating the bag **202** such as with its handles. This pressure produces a reaction force from the urethra opening, indicated by the force arrows **230** in Figure 16A , which collapses the extension **226** of the bag **202.** Contact of the collapsing extension **226** pivots the locking tab **222** toward or against the main body **224** such that the orifice becomes aligned with the catheter axis - the unlocked position. At this stage, manual coordinated movement of the first and second movement control devices **206 , 208** by grasping through the bag **202** advances the catheter **204** from within the bag. Since the catheter **204** emerges directly into the urethra by virtue of the pre-inserted introducer tip **212** , no bacteria is carried into the urethra.

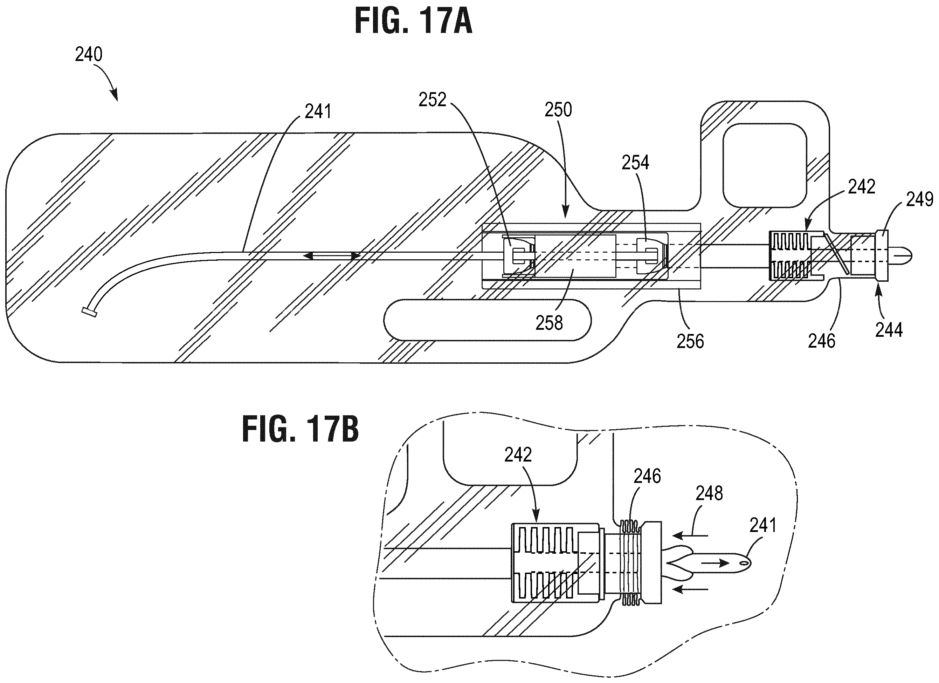

Figure 17A is a front view of another embodiment of a packaged catheter **240** having a catheter **241** housed within a sterile bag with an outlet end **244.** As with the sterile catheter package **200,** a safety device in the form of a movement control device **242** mounts within the sterile bag close to the outlet **244,** and in particular just inside a collapsible extension **246** of the bag. The movement control device **242** works in the same manner as described above. Figure 17B shows collapse of the extension **246** of the bag from reaction pressure **248** against the urethra such that the movement control device **242** opens or unlocks and the catheter **241** may be advanced. In this embodiment, the outlet **244** features a solid cap member **249** having an inwardly-extending tubular portion that contacts the locking tab of the movement control device **242.**

The packaged catheter **240** also has an integrated assembly **250** of a pair of movement control devices **252 , 254** mounted therein for advancing a catheter. The integrated assembly **250** may be the same as the integrated assembly **120** described above with respect to Figure 11 , and includes a larger base member **256** and a smaller movable member **258** arranged to slide within the base member. The smaller movable member **258** has a finger pad on its front surface that may be manipulated through the flexible bag, or the finger pad may be adhered to an inner side of the outer wall of the bag. Reciprocal sliding of the movable member **258** within the base member **256** advances the catheter **241** out of the outlet **244** of the sterile bag.

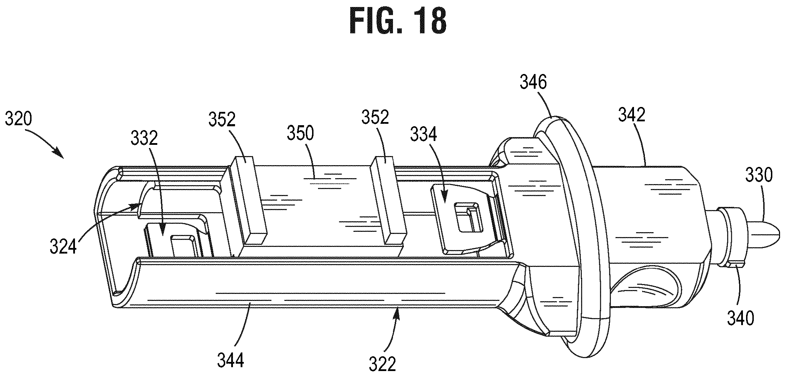

Figure 18 is a perspective view of an integrated assembly **320** of a pair of movement control devices within relatively sliding housings which is similar to the integrated assembly **120** described above within respect to Figure 12. The integrated assembly **320** includes a larger base member **322** and a smaller movable member **324** arranged to move within the base member. The base member **322** may be secured to one end of a sterile bag, such as schematically shown at 126 in Figure 11 , and defines an exterior handle grip of the integrated assembly **320.** A catheter (not shown) passes longitudinally through the assembly **320** and typically is positioned with its distal tip just inside of an introducer tip **330** in a stored position.

A first or inner movement control device **332** forms a part of the movable member **324** while a second or outer movement control device **334** forms a part of the base member **322.** It will be understood that relative displacement of the movement control devices **332 , 334** advances the catheter in the "inchworm" manner depicted in Figures 10A-10C .

As in the detailed views of the prior embodiment of Figures 12-15 , the larger base member **322** includes a distal hub **340** from which the introducer tip **330** projects. The hub **340** in turn is preferably molded together with an ergonomic external handle **342** and an internal housing **344** which are separated by a flange **346.** When assembled with the sterile bag, the handle **342** is outside while the housing **344** is inside, with the bag preferably adhered or heat sealed to the exterior of the flange **346 ,** such as seen in Figure 11 .

The movable member **324** has a generally tubular housing topped by an upper finger pad **350.** The upper finger pad **350** has an alternative configuration than flat as shown, with two spaced apart and laterally-extending raised bars **352** on an upper surface. The bars **352** in conjunction with the recessed area therebetween facilitates purchase of a person's finger, thumb, or other portion of the hand or arm when sliding the movable member **124** back-and-forth through the bag. That is, the bars **352** project upward and thus form an easy-to-manipulate finger pad 350. Of course, other such projections upward from a flat finger pad may be used, such as an "X" pattern or the like. The finger pad **350** is manipulated by the user through the flexible bag, and may also be adhered to an inner surface of the bag to avoid slippage therebetween.

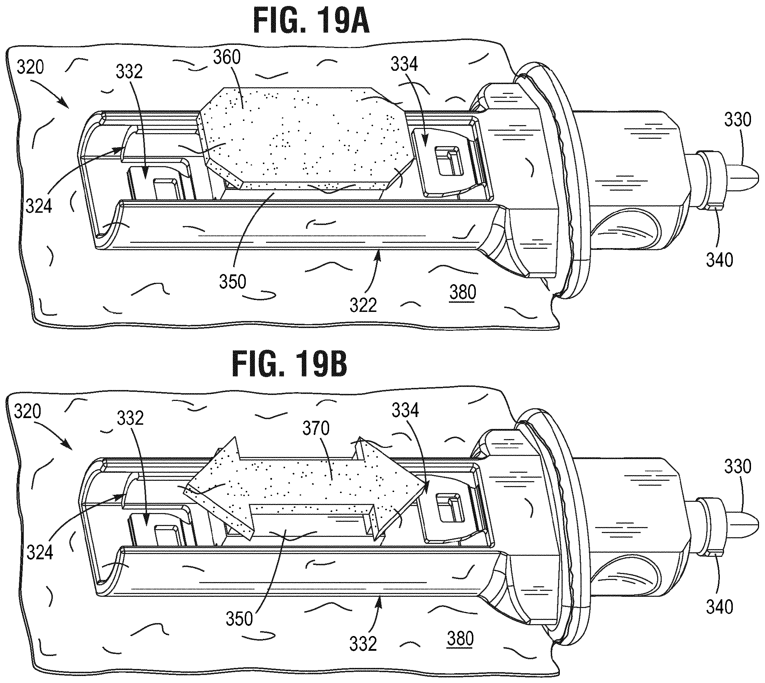

Figures 19A and 19B are perspective views of the integrated assembly **320** of Figure 18 mounted to a sterile bag with alternative friction-enhancing pads **360 , 370** in operative relationship with the finger pad **350.** The friction-enhancing pads **360 , 370** are desirably adhered to an outer panel **380** of the bag directly over the finger pad **350.** The friction-enhancing pad **360** of Figure 19A has a six-sided polygonal shape which maximizes surface area over the finger pad **350** , while the friction-enhancing pad **370** of Figure 19B has a double-headed arrow shape to indicate the direction of sliding movement. Alternatively, a double-headed arrow may be printed onto the polygonal friction-enhancing pad **360.** The friction-enhancing pads **360 , 370** may be made of various compressible materials, such as rubber, closed-cell foam, or the like, or may be formed of a plastic with friction-enhancing features provided on the upper surface, such as bumps, ribs, a gritty layer, or others. Preferably the friction-enhancing pads **360 , 370** are flexible to conform to the bag and assist the user in transmitting force to the underlying finger pad **350.** As before, the finger pad **350** may be adhered to an inner surface of the bag to avoid slippage therebetween.

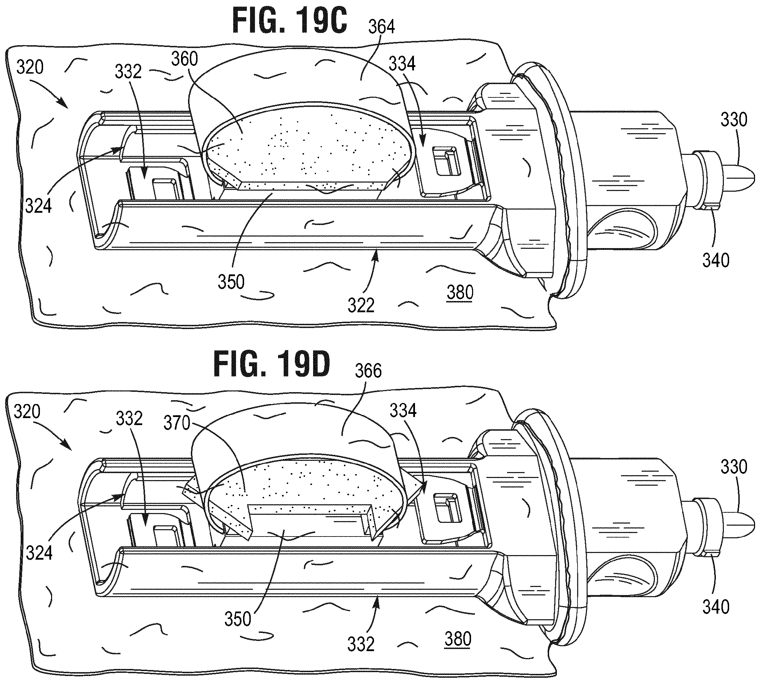

Figures 19C and 19D are perspective views of the integrated assembly **320** of Figure 18 mounted to a sterile bag with the alternative friction-enhancing pads **360 , 370** supplemented with loops or straps **364 , 366.** The straps **364, 366** may be used to help a user grasp and linearly reciprocate the finger pad **350.**

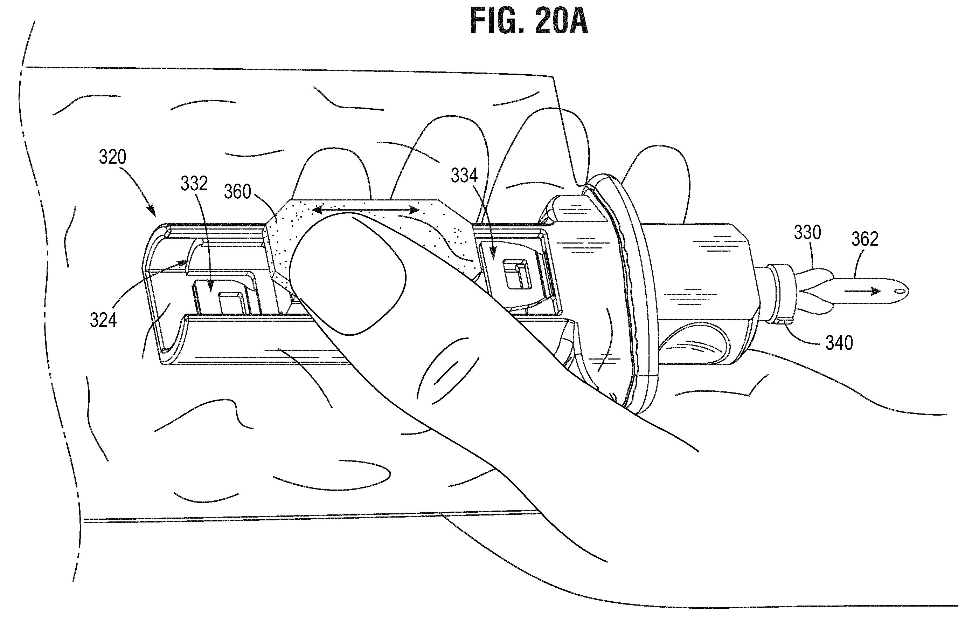

Figures 20A-20D are perspective views of the integrated assemblies **320** of Figures 19A and 19C mounted to an outer panel of a sterile bag showing a variety of different ways to grasp and reciprocate the finger pad **350** to advance the catheter from within the bag. For instance, Figure 20A shows a user holding the outlet end of the bag in one hand with the palm and fingers under the integrated assembly **320** and the thumb in contact with the friction-enhancing pad **360.** Back-and-forth movement of the thumb on the friction-enhancing pad **360** advances the catheter **362** out of the sterile bag.

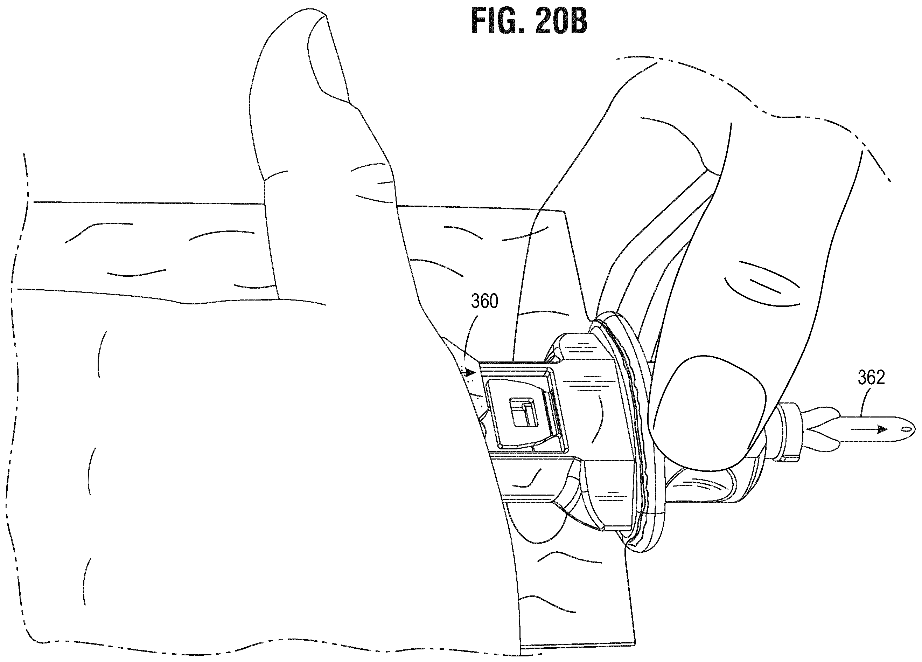

Figure 20B shows a two-handed operation, where the right hand grasps and steadies the integrated assembly **320** while the left hand reciprocates the friction-enhancing pad **360** and underlying finger pad **350.** Some users have a difficult time with dexterity, and so the user may contact the wide friction-enhancing pad **360** with the heel of his or her palm.

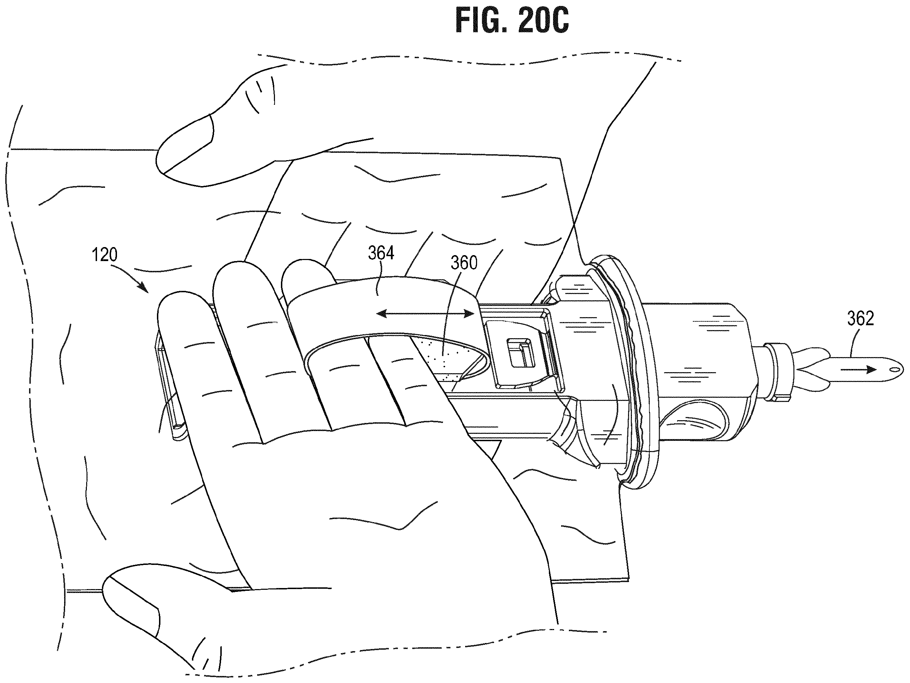

In Figure 20C the user merely sandwiches the integrated assembly **320** between his or her two hands, without grasping at all. One or more fingers may be inserted through the strap **364** to gain purchase on the finger pad **350** without the need for grasping. Back-and-forth movement of the hand in contact with the friction-enhancing pad **360** and within the strap **364** advances the catheter **362.**

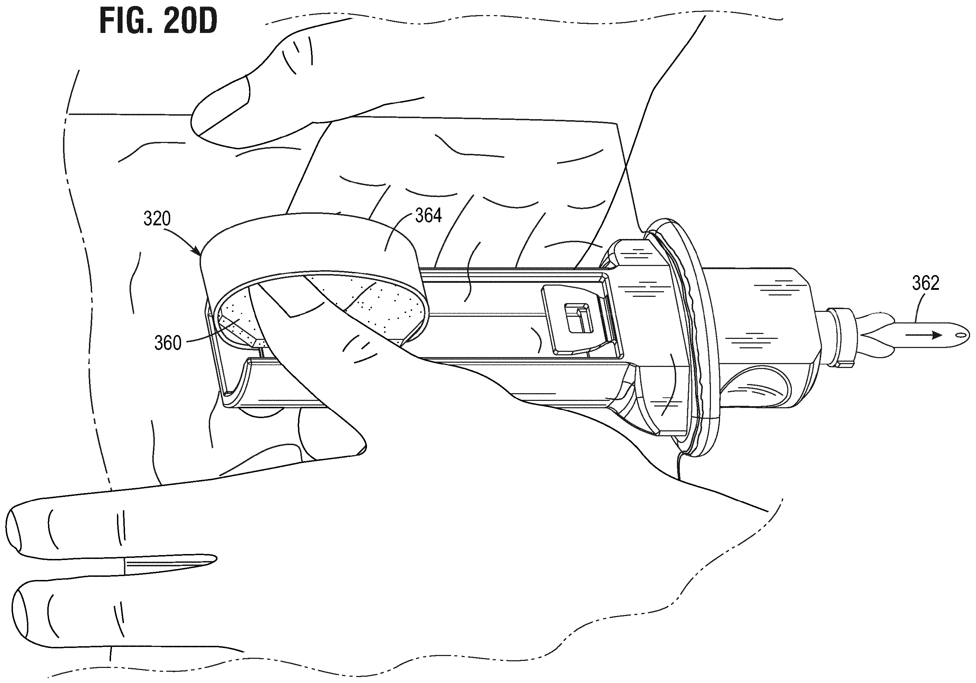

Finally, Figure 20D illustrates a still further possible configuration where the user stabilizes the integrated assembly **320** from underneath with the right hand while manipulating the friction-enhancing pad **360** with the thumb of the left hand through the strap **364.** It should be noted again that the generally rectangular lateral cross-section of the integrated assembly **320** aids in these various movements by presenting resistance to rotation about the longitudinal axis and also a flatter profile for the user to compress between his or her hands.

Throughout this description, the embodiments and examples shown should be considered as exemplars, rather than limitations on the apparatus and procedures disclosed or claimed. Although many of the examples presented herein involve specific combinations of method acts or system elements, it should be understood that those acts and those elements may be combined in other ways to accomplish the same objectives. Acts, elements and features discussed only in connection with one embodiment are not intended to be excluded from a similar role in other embodiments.

## Claims

1. A closed intermittent urinary catheter system equipped with a catheter dispensing device, comprising:
a) sterile flexible packaging (20) defining a closed reservoir with an outlet (210) opening;
b) a catheter (60) defining a longitudinal axis (69CA) retained within the reservoir and having a distal insertion tip (61) positioned adjacent to and aimed toward the outlet (210) opening;
c) a first movement control device (31) mounted over the catheter (60) proximally from the distal insertion tip (61) and manually manipulable from outside and through the flexible packaging (20), the first movement control device (61) having a locking member (50) that moves to an unlocked position upon relative distal movement of the catheter (60) therein to allow relative distal movement of the catheter (60), and moves to a locked position upon relative proximal movement of the catheter (60) to prevent further relative proximal movement of the catheter (60); and
d) a second movement control device (32) mounted over the catheter (60) between the distal insertion tip (61) and the first movement control device (31) and being manually manipulable from outside and through the flexible packaging (20), the second movement control device (32) having a locking member (50) that moves to an unlocked position upon relative distal movement of the catheter (60) therein to allow relative distal movement of the catheter (60), and moves to a locked position upon relative proximal movement of the catheter (60) to prevent further relative proximal movement of the catheter (60), wherein
e) coordinated movement of the first and second movement control devices (31, 32) toward and away from one another causes the catheter (60) to be advanced distally through the outlet (210) opening; and
f) a third movement control device (220) disposed at and attached to one end of the flexible packaging (20) just inside the outlet (210) opening, the third movement control device (220) having a locking member (50) that is biased to a locked position that prevents relative distal movement of the catheter (60) through the third movement control device (220), and wherein the outlet (210) opening of the flexible packaging (50) has an introducer tip (114) sized to fit within the outer end of the urethra and made of a flexible elastomer which has petals that the catheter (60) spreads apart upon distal passage therethrough, and the packaging is collapsible at the outlet (210) opening so that pressure on the introducer tip (114) from the urethra collapses the packaging (220) and moves the locking member (50) from the locked position to an unlocked position that allows relative distal movement of the catheter (60) through the third movement control device (220).

2. The system of claim 1, further including a handle grip (70) that includes (i) a hand graspable base member (71) fixedly attached to the second movement control device (32), and (ii) a movable member (72) reciprocally engaged to the base member (71) and fixedly attached to the first movement control device (32), wherein reciprocation of the movable member (72) relative to the base member (71) effects the coordinated movement of the first and second movement control devices (31, 32) so as to effect distal advancement of the catheter (60) through the outlet (210) opening.

3. The system of claim 2, wherein the movable member (72) is slidably disposed for reciprocal movement in a distal-proximal direction within a channel in the base member (71), the movable member (72) including a finger pad (174) facing toward the flexible packaging (20) that is manually manipulable from outside and through the flexible packaging (20), the finger pad (174) having a flat surface.

4. The system of claim 3, wherein the finger pad (174) further includes two laterally-oriented bars (352) on opposite longitudinal ends of the flat surface.

5. The system of claim 3, further including a friction-enhancing pad adhered to an outer surface of the flexible packaging directly outside of the finger pad.

6. The system of claim 5, wherein the friction-enhancing pad (360) is a compressible material.

7. The system of claim 5, wherein the friction-enhancing pad (360) has a double-headed arrow indicator thereon to indicate the distal-proximal reciprocal movement direction of the movable member (72).

8. The system of claim 3, wherein the finger pad (360) is adhered to the flexible packaging (20) and further including a strap (364) attached outside the flexible packaging (20) directly outside of the finger pad (360), the strap (364) sized to receive one or more fingers of a user.

9. The system of any preceding claim, wherein the flexible packaging (20) defines a collapsible extension (226) at the outlet (210) opening and the third movement control device (220) includes a main body (40) having a passageway (49) defining a central axis (49CA), and the locking member (50) is a locking tab (222) hingedly attached to the main body (50), and the locking tab (222) is located within the collapsible extension (226) such that pressure on the introducer tip from the urethra collapses the collapsible extension (226) and moves the locking tab (222) from the locked position to the unlocked position that allows relative distal movement of the catheter (60) through the third movement control device (202).

10. The system of claim 9, wherein the outlet (210) opening has a solid cap member (249) having an inwardly-extending tubular portion that contacts the locking tab (222) of the third movement control device (220) when the collapsible extension (226) collapses.

11. The system of any preceding claim, wherein:
a) each movement control device (31, 32, 220) comprises (i) a main body (40) having a passageway (49) defining a central axis (49CA), and (ii) the locking member (50) hingedly attached to the main body (40) for pivoting about a pivot axis (55P) and having an orifice (59) defining a central axis (59CA) extending therethrough, (iii) whereby the locking member (50) is pivotable between the unlocked position wherein the central axis (59CA) of the orifice (59) is aligned with the central axis (49CA) of the passageway (49) and permits distal movement of the catheter (60) relative to the movement control device (31, 32, 220), and the locked position wherein the central axis (59CA) of the orifice (59) is misaligned with the central axis (49CA) of the passageway (49) and resists proximal movement of the catheter (60) relative to the movement control device (31, 32, 220), and
b) the catheter (60) extends through the passageway (49) and the orifice (59) in both of the movement control devices (31, 32, 220).

12. The system of claim 11, further comprising a release element within the reservoir in operable engagement with the locking member (50) of the first movement control device (31) and manipulable from outside and through the flexible packaging (20), the release element configured to be actuated and hold the locking members (50) of the first and second movement control devices (31, 32) in their unlocked positions so as to permit free movement of the catheter (60) through the first and second movement control devices (31, 32).

13. The system of claim 11, wherein the main body (40) of each of the first and second movement control devices (31, 32) has longitudinally (x) spaced opposed first and second ends (41, 42), and the passageway (49) through the main body (40) extends between an opening (49i) in the first end (41) of the main body (40) and an opening (49e) in the second end (42) of the main body (40), and first and second laterally (y) spaced engagement members (43) extend longitudinally (x) from the first end (41) of the main body (40), the engagement members (43) positioned on diametric sides of the opening (49i) in the first end (41) of the main body (40), and the locking member (50) is hingedly attached at a first lateral end (51) to the first engagement member (431) for pivoting about the pivot axis (55p).

## Patentansprüche

1. Geschlossenes intermittierendes Harnkathetersystem, das mit einer Katheterausgabevorrichtung ausgestattet ist, aufweisend:
a) eine sterile flexible Packung (20), die ein geschlossenes Reservoir mit einer Auslassöffnung (210) definiert;
b) einen Katheter (60), der eine Längsachse (69CA) definiert und innerhalb des Reservoirs gehalten wird und mit einer distalen Einsatzspitze (61) versehen ist, die benachbart zur Auslassöffnung (210) positioniert ist und auf diese gerichtet ist;
c) eine erste Bewegungssteuerungsvorrichtung (31), die proximal von der distalen Einsatzspitze (61) über dem Katheter (60) angebracht ist und von außen und durch die flexible Packung (20) manuell bedienbar ist, wobei die erste Bewegungssteuerungsvorrichtung (61) ein Verriegelungselement (50) aufweist, das sich bei einer relativen distalen Bewegung des Katheters (60) darin in eine entriegelte Position bewegt, um eine relative distale Bewegung des Katheters (60) zu ermöglichen, und sich bei einer relativen proximalen Bewegung des Katheters (60) in eine verriegelte Position bewegt, um eine weitere relative proximale Bewegung des Katheters (60) zu verhindern; und
d) eine zweite Bewegungssteuerungsvorrichtung (32), die über dem Katheter (60) zwischen der distalen Einsatzspitze (61) und der ersten Bewegungssteuerungsvorrichtung (31) angebracht ist und von außen und durch die flexible Packung (20) manuell bedienbar ist, wobei die zweite Bewegungssteuerungsvorrichtung (32) ein Verriegelungselement (50) aufweist, das sich bei einer relativen distalen Bewegung des Katheters (60) darin in eine entriegelte Position bewegt, um eine relative distale Bewegung des Katheters (60) zu ermöglichen, und sich bei einer relativen proximalen Bewegung des Katheters (60) in eine verriegelte Position bewegt, um eine weitere relative proximale Bewegung des Katheters (60) zu verhindern, wobei
e) eine koordinierte Bewegung der ersten und zweiten Bewegungssteuerungsvorrichtung (31, 32) aufeinander zu und voneinander weg bewirkt, dass der Katheter (60) distal durch die Auslassöffnung (210) vorgeschoben wird; und
f) eine dritte Bewegungssteuerungsvorrichtung (220), die an einem Ende der flexiblen Packung (20) direkt innerhalb der Auslassöffnung (210) angeordnet und daran befestigt ist, wobei die dritte Bewegungssteuerungsvorrichtung (220) ein Verriegelungselement (50) aufweist, das in eine verriegelte Position vorgespannt ist, die eine relative distale Bewegung des Katheters (60) durch die dritte Bewegungssteuerungsvorrichtung (220) verhindert, und wobei die Auslassöffnung (210) der flexiblen Packung (50) eine Einführspitze (114) aufweist, die so dimensioniert ist, dass sie in das äußere Ende der Harnröhre passt, und aus einem flexiblen Elastomer besteht, das Lamellen aufweist, die der Katheter (60) beim distalen Durchgang auseinander spreizt, und wobei die Packung an der Auslassöffnung (210) zusammenklappbar ist, so dass Druck auf die Einführspitze (114) aus der Harnröhre die Packung (220) zusammenfallen lässt und das Verriegelungselement (50) aus der verriegelten Position in eine entriegelte Position bewegt, die eine relative distale Bewegung des Katheters (60) durch die dritte Bewegungssteuerungsvorrichtung (220) ermöglicht.

2. System nach Anspruch 1, das ferner einen Griff (70) umfasst, der (i) ein mit der Hand greifbares Grundelement (71), das fest mit der zweiten Bewegungssteuerungsvorrichtung (32) verbunden ist, und (ii) ein bewegliches Element (72), das mit dem Grundelement (71) in Wechselwirkung steht und fest an der ersten Bewegungssteuerungsvorrichtung (32) befestigt ist, umfasst, wobei die Hin- und Herbewegung des beweglichen Elements (72) relativ zu dem Grundelement (71) die koordinierte Bewegung der ersten und zweiten Bewegungssteuerungsvorrichtung (31, 32) bewirkt, um ein distales Vorschieben des Katheters (60) durch die Auslassöffnung (210) zu bewirken.

3. System nach Anspruch 2, wobei das bewegliche Element (72) für eine Hin- und Herbewegung in distaler-proximaler Richtung innerhalb eines Kanals in dem Grundelement (71) verschiebbar angeordnet ist, wobei das bewegliche Element (72) eine Fingerauflage (174) umfasst, die zur flexiblen Packung (20) hin ausgerichtet ist und von außen und durch die flexible Packung (20) hindurch manuell bedienbar ist, wobei die Fingerauflage (174) eine flache Oberfläche aufweist.

4. System nach Anspruch 3, wobei die Fingerauflage (174) ferner zwei seitlich ausgerichtete Stangen (352) an gegenüberliegenden Längsenden der flachen Oberfläche aufweist.

5. System nach Anspruch 3, das ferner eine reibungserhöhende Auflage umfasst, die direkt außerhalb der Fingerauflage an einer Außenfläche der flexiblen Packung haftet.

6. System nach Anspruch 5, wobei die reibungserhöhende Auflage (360) aus einem komprimierbaren Material besteht.

7. System nach Anspruch 5, wobei die reibungserhöhende Auflage (360) mit einer Doppelpfeilanzeige versehen ist, um die distale-proximale Hin- und Herbewegungsrichtung des beweglichen Elements (72) anzuzeigen.

8. System nach Anspruch 3, wobei das Fingerpolster (360) an der flexiblen Packung (20) befestigt ist und ferner einen Gurt (364) umfasst, der außerhalb der flexiblen Packung (20) direkt außerhalb des Fingerpolsters (360) angebracht ist, wobei der Gurt (364) so dimensioniert ist, dass er einen oder mehrere Finger eines Benutzers aufnehmen kann.

9. System nach einem der vorhergehenden Ansprüche, wobei die flexible Packung (20) eine zusammenklappbare Verlängerung (226) an der Auslassöffnung (210) definiert und die dritte Bewegungssteuerungsvorrichtung (220) einen Hauptkörper (40) umfasst, der einen Durchgang (49) aufweist, der eine Mittelachse (49CA) definiert, und das Verriegelungselement (50) eine Verriegelungslasche (222) ist, die schwenkbar am Hauptkörper (50) angebracht ist, und die Verriegelungslasche (222) innerhalb der zusammenklappbaren Verlängerung (226) angeordnet ist, so dass Druck auf die Einführspitze aus der Harnröhre die zusammenklappbare Verlängerung (226) zusammenklappt und die Verriegelungslasche (222) aus der verriegelten Position in die entriegelte Position bewegt, die eine relative distale Bewegung des Katheters (60) durch die dritte Bewegungssteuerungsvorrichtung (202) ermöglicht.

10. System nach Anspruch 9, wobei die Auslassöffnung (210) ein festes Kappenelement (249) mit einem nach innen ragenden röhrenförmigen Abschnitt aufweist, der mit der Verriegelungslasche (222) der dritten Bewegungssteuerungsvorrichtung (220) in Kontakt kommt, wenn die zusammenklappbare Verlängerung (226) zusammenklappt.

11. System nach einem der vorhergehenden Ansprüche, wobei:
a) jede Bewegungssteuerungsvorrichtung (31, 32, 220) (i) einen Hauptkörper (40) mit einem Durchgang (49), der eine Mittelachse (49CA) definiert, und (ii) das Verriegelungselement (50), das schwenkbar am Hauptkörper (40) angebracht ist, um es um eine Drehachse (55P) zu schwenken, und ein Durchlass (59) aufweist, der eine sich durch diesen erstreckende Mittelachse (59CA) definiert, aufweist und (iii) wobei das Verriegelungselement (50) zwischen der entriegelten Position, in der die Mittelachse (59CA) des Durchlasses (59) mit der Mittelachse (49CA) des Durchgangs (49) fluchtet und eine distale Bewegung des Katheters (60) relativ zu der Bewegungssteuerungsvorrichtung (31, 32, 220) ermöglicht, und der verriegelten Position, wobei die Mittelachse (59CA) des Durchlasses (59) nicht mit der Mittelachse (49CA) des Durchgangs (49) fluchtet und einer proximalen Bewegung des Katheters (60) relativ zu der Bewegungssteuerungsvorrichtung (31, 32, 220) Widerstand leistet, und
b) sich der Katheter (60) durch den Durchgang (49) und den Durchlass (59) in beiden Bewegungssteuerungsvorrichtungen (31, 32, 220) erstreckt.

12. System nach Anspruch 11, das ferner ein Auslöseelement innerhalb des Reservoirs aufweist, das in betätigbarer Verbindung mit dem Verriegelungselement (50) der ersten Bewegungssteuerungsvorrichtung (31) steht und von außen und durch die flexible Packung (20) hindurch betätigbar ist, wobei das Auslöseelement so ausgebildet ist, dass es betätigt werden kann und die Verriegelungselemente (50) der ersten und zweiten Bewegungssteuerungsvorrichtung (31, 32) in ihrer entriegelten Position hält, um eine freie Bewegung des Katheters (60) durch die erste und zweite Bewegungssteuerungsvorrichtung (31, 32) zu ermöglichen.

13. System nach Anspruch 11, wobei der Hauptkörper (40) jeder der ersten und zweiten Bewegungssteuerungsvorrichtungen (31, 32) in Längsrichtung (x) beabstandete gegenüberliegende erste und zweite Enden (41, 42) aufweist und sich der Durchgang (49) durch den Hauptkörper (40) zwischen einer Öffnung (49i) in dem ersten Ende (41) des Hauptkörpers (40) und einer Öffnung (49e) in dem zweiten Ende (42) des Hauptkörpers (40) erstreckt, und sich ein erstes und ein zweites seitlich (y) beabstandetes Eingriffselement (43) in Längsrichtung (x) von dem ersten Ende (41) des Hauptkörpers (40) erstrecken, wobei die Eingriffselemente (43) auf diametralen Seiten der Öffnung (49i) in dem ersten Ende (41) des Hauptkörpers (40) positioniert sind und das Verriegelungselement (50) an einem ersten seitlichen Ende (51) schwenkbar an dem ersten Eingriffselement (431) befestigt ist, um es um die Drehachse (55p) zu schwenken.

## Revendications

1. Un système fermé de cathéter urinaire intermittent équipé d'un dispositif de distribution de cathéter, comprenant :
a) un emballage (20) stérile souple définissant un réservoir fermé ayant une ouverture de sortie (210) ;
b) un cathéter (60) définissant un axe (69CA) longitudinal, retenu dans le réservoir, ayant une pointe (61) distale d'insertion en position au voisinage de l'ouverture de sortie (210) et pointant vers celle-ci ;
c) un premier dispositif (31) de commande de déplacement monté sur le cathéter (60) proximalement à la pointe (61) distale d'insertion et manipulable manuellement de l'extérieur et traversant l'emballage (20) souple, le premier dispositif (61) de commande de déplacement ayant un élément (50) de verrouillage, qui vient dans une position déverrouillée après que le cathéter (60) s'y déplace suivant un déplacement distal relatif, pour permettre un déplacement distal relatif du cathéter (60), et vient dans une position verrouillée, après déplacement proximal relatif du cathéter (60), pour empêcher le cathéter (60) d'avoir davantage de déplacement proximal relatif ; et
d) un deuxième dispositif (32) de commande de déplacement monté sur le cathéter (60) entre la pointe (61) distale d'insertion et le premier dispositif (31) de commande de mouvement et manipulable manuellement de l'extérieur et traversant l'emballage (20) souple, le deuxième dispositif (32) de commande de mouvement ayant un élément (50) de verrouillage, qui vient dans une position déverrouillée, après que le cathéter (60) s'y déplace suivant un mouvement distal relatif, pour permettre un mouvement distal relatif du cathéter (60), et vient dans une position verrouillée après mouvement proximal relatif du cathéter (60), pour empêcher le cathéter (60) de se déplacer davantage suivant un déplacement proximal relatif, dans lequel
e) un déplacement coordonné du premier et du deuxième dispositifs (31, 32) de commande de déplacement les rapprochant et les éloignant l'un de l'autre fait que le cathéter (60) est avancé distalement dans l'ouverture de sortie (210) ; et
f) un troisième dispositif (220) de commande de déplacement disposé à et fixé à une extrémité de l'emballage (20) souple juste à l'intérieur de l'ouverture de sortie (210), le troisième dispositif (220) de commande de mouvement ayant un élément (50) de verrouillage, qui est sollicité vers une position verrouillée pour empêcher un mouvement distal relatif du cathéter (60) par le troisième dispositif (220) de commande de mouvement et dans lequel l'ouverture de sortie (210) de l'emballage (50) souple a un bout (114) d'introducteur dimensionné pour s'adapter dans l'extrémité extérieure de l'urètre et fait d'un élastomère souple, qui a des pétales que le cathéter (60) étale après y avoir passé distalement et l'emballage peut s'affaisser à l'ouverture de sortie (210), de manière à ce que de la pression sur la pointe (114) de l'introducteur par l'urètre affaisse l'emballage (220) et fasse passer l'élément (50) de verrouillage de la position verrouillée à une position déverrouillée, qui permet un mouvement distal relatif du cathéter (60) dans le troisième dispositif (220) de commande de mouvement.

2. Le système de la revendication 1, comprenant en outre une poignée (70) de manipulation, qui comprend (i) un élément (71) de base préhensible, fixé de manière fixe au deuxième dispositif (32) de commande de déplacement et (ii) un élément (72) mobile enclenché réciproquement dans l'élément (71) de base et fixé de manière fixe au premier dispositif (32) de commande de déplacement, dans lequel la réciprocité de l'élément (72) mobile par rapport à l'élément (71) de base effectue le déplacement coordonné des premier et deuxième dispositifs (31, 32) de commande de mouvement, de manière à effectuer un avancement distal du cathéter (60) dans l'ouverture de la sortie (210).

3. Le système de la revendication 2, dans lequel l'élément (72) mobile est monté coulissant pour un déplacement réciproque dans une direction distale-proximale dans un canal de l'élément (71) de base, l'élément (72) mobile comprenant un tampon (174) à doigt faisant face à l'emballage (20) souple, qui est manipulable manuellement de l'extérieur et traversant l'emballage (20) souple, le tampon (174) à doigt ayant une surface plane.

4. Le système de la revendication 3, dans lequel le tampon (174) à doigt comprend en outre deux barres (352) orientées latéralement sur des extrémités longitudinales opposées de la surface plane.

5. Le système de la revendication 3, comprenant en outre un tampon augmentant le frottement, adhérent à une surface extérieure de l'emballage souple directement à l'extérieur du tampon à doigt.

6. Le système de la revendication 5, dans lequel le tampon (360) augmentant le frottement est un matériau compressible.

7. Le système de la revendication 5, dans lequel le tampon (360) augmentant le frottement a sur lui un indicateur par flèche à deux têtes pour indiquer la direction du mouvement réciproque distale-proximale de l'élément (72) mobile.

8. Le système de la revendication 3, dans lequel le tampon (360) à doigt adhère à l'emballage (20) souple et comprend en outre une bande (364) fixée à l'extérieur de l'emballage (20) souple directement à l'extérieur du tampon (360) à doigt, la bande (364) étant dimensionnée pour recevoir un ou plusieurs doigts d'un utilisateur.

9. Le système de l'une quelconque des revendications précédentes, dans lequel l'emballage (20) souple définit un prolongement (226) pouvant s'affaisser à l'ouverture de sortie (210) et le troisième dispositif (220) de commande de déplacement comprend un corps (40) principal ayant un passage (49) définissant un axe (49CA) central, et l'élément (50) de verrouillage est un ergot (222) de verrouillage fixé à charnière au corps (50) principal et l'ergot (222) de verrouillage est disposé dans le prolongement (226) pouvant s'affaisser, de manière que de la pression sur la pointe de l'introducteur par l'urètre affaisse le prolongement (226) pouvant s'affaisser et fasse passer l'ergot (222) de verrouillage de la position verrouillée à la position déverrouillée, qui autorise un déplacement distal relatif du cathéter (60) dans le troisième dispositif (202) de commande de déplacement.

10. Le système de la revendication 9, dans lequel l'ouverture de sortie (210) a un élément (249) plein de capuchon ayant une partie tubulaire s'étendant vers l'intérieur, qui est en contact avec l'ergot (222) de verrouillage du troisième dispositif (220) de commande de déplacement, lorsque le prolongement (226), qui peut s'affaisser, s'affaisse.

11. Le système de l'une quelconque des revendications précédentes, dans lequel :
a) chaque dispositif (31, 32, 220) de commande de déplacement comprend (i) un corps (40) principal ayant le passage (49) définissant un axe (49CA) central et (ii) l'élément (50) de verrouillage fixé à charnière au corps (40) principal pour pivoter autour d'un axe (55P) de pivotement ayant un orifice (59) définissant un axe (59CA) central, s'y étendant (iii), l'élément (50) de verrouillage pouvant ainsi pivoter entre la position déverrouillée, dans laquelle l'axe (59CA) central de l'orifice (59) est aligné sur l'axe (49CA) central du passage (49) et permet un déplacement distal du cathéter (60) par rapport au dispositif (31, 32, 220) de commande de déplacement, et la position verrouillée, dans laquelle l'axe (59CA) central de l'orifice (59) est désaligné de l'axe (49CA) central du passage (49) et résiste à un déplacement proximal du cathéter (60) par rapport au dispositif (31, 32, 220) de commande de mouvement, et
b) le cathéter (60) s'étend dans le passage (49) et l'orifice (59) dans à la fois les dispositifs (31, 32, 220) de commande de déplacement.

12. Le système de la revendication 11, comprenant en outre un élément de libération dans le réservoir en coopération de fonctionnement avec l'élément (50) de verrouillage du premier dispositif (31) de commande de mouvement et manipulable de l'extérieur et traversant l'emballage (20) souple, l'élément de libération étant configuré pour être actionné et maintenir les éléments (50) de verrouillage du premier et du deuxième dispositifs (31, 32) de commande de déplacement dans leurs positions déverrouillées, de manière à permettre un déplacement libre du cathéter (60) dans le premier et le deuxième dispositifs (31, 32) de commande de mouvement.

13. Le système de la revendication 11, dans lequel le corps (40) principal de chacun du premier et du deuxième dispositifs (31, 32) de commande de mouvement a des première et deuxième extrémités (41, 42) opposées à distance longitudinalement (x) et le passage (49) dans le corps (40) principal s'étend entre une ouverture (49i) dans la première extrémité (41) du corps (40) principal et une ouverture (49e) dans la deuxième extrémité (42) du corps (40) principal et des premier et deuxième éléments (43) d'enclenchement à distance latéralement (y) s'étendent longitudinalement (x) à partir de la première extrémité (41) du corps (40) principal, les éléments (43) d'enclenchement étant en position sur des faces diamétrales de l'ouverture (49i) dans la première extrémité (41) du corps (40) principal et l'élément (50) de verrouillage est fixé à charnière à une première extrémité (51) latérale au premier élément (431) d'enclenchement pour pivoter autour de l'axe (55p) de pivotement.
